(19) Europäisches Patentamt
European Patent Office
Office européen des brevets

(11) Publication number: **0 194 077**
**A1**

(12) # EUROPEAN PATENT APPLICATION

(21) Application number: **86301205.0**

(22) Date of filing: **20.02.86**

(51) Int. Cl.⁴: **A 01 N 43/16**, C 07 D 309/30

(30) Priority: **21.02.85 GB 8504483**

(43) Date of publication of application: **10.09.86 Bulletin 86/37**

(84) Designated Contracting States: **AT BE CH DE FR GB IT LI LU NL SE**

(71) Applicant: **NATIONAL RESEARCH DEVELOPMENT CORPORATION, 101 Newington Causeway, London SE1 6BU (GB)**

(72) Inventor: **Briggs, Geoffrey Gower, 46 Granby Avenue, Harpenden Hertfordshire AL5 5QR (GB)**
Inventor: **Cayley, George Raymond, 32 Topstreet Way, Harpenden Hertfordshire AL5 5TT (GB)**
Inventor: **Laurence, Brian Robin, 17a Colebrooke Row, London N1 8DB (GB)**
Inventor: **Pickett, John Anthony, 53 Parkfield Crescent, Kimpton, Hitchin Hertfordshire SG4 8EQ (GB)**

(74) Representative: **Stephenson, Gerald Frederick, Patent Department National Research Development Corporation 101 Newington Causeway, London SE1 6BU (GB)**

(54) **Improvements in or relating to pheromones.**

(57) Compounds of formula (II)

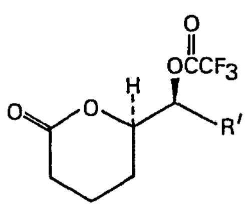

(II)

in which R′ represents an alkyl group of 6 to 12 carbon atoms, are of value as mosquito attractants, mosquitoes being attracted to a location where they and/or their eggs or larvae are then destroyed.

EP 0 194 077 A1

0194077



127872

# IMPROVEMENTS IN OR RELATING TO PHEROMONES

This invention relates to compounds having activity as oviposition attractants for mosquitoes and to the use of these compounds in mosquito control.

The control of mosquitoes using mosquito pheromones is of interest in view of the involvement of mosquitoes of the genus Culex in the spread of a number of diseases in various parts of the world, for example Culex pipiens fatigans acting as a vector for filarial disease (Wucheria bancrofti) known as elephantiasis, Culex tarsalis for Western equine encephalitis, the Culex tritaeniorhynchus group for Japanese B encephalitis and other viral diseases, and the Culex pipiens groups for Rift Valley fever. The attraction of mosquitos of the genus Culex such as Culex pipiens pipiens, Culex pipiens molestus, Culex tarsalis and particularly Culex pipiens fatigans (= quinquefasciatus) to their own egg rafts has been recognised for some time, certain glycerides having been proposed as the source of this attaction. Recently, however, novel mosquito pheromones of a non-glyceride type have been found by two of us in the apical droplets of eggs of Culex pipiens fatigans.

These non-glyceride pheromones have been identified as 6-acetoxy-5-tetradecanolide and 6-acetoxy-5-hexadecanolide [formula (I), R = acetyl and R' = n-octyl or n-decyl, respectively]

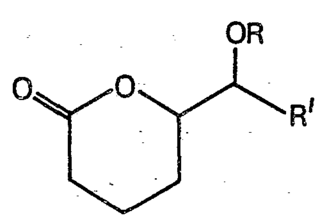

(I)

and are the subject of UK Patent No. 2111481 which also describes various analogues of the pheromones that vary in the nature of the group R and/or the group R'. However, although these analogues

are of interest as alternatives to the compounds which occur in nature, it should be stressed that the tests which have been carried out on the compounds which are the subject of that patent have shown 6-acetoxy-5-hexadecanolide to be the most active of these compounds. We have now found, however, that one particular form of modification of the compound 6-acetoxy-5-hexadecanolide does not cause a reduction in activity and that, surprisingly, the modified compound shows a similar or even higher level of activity than the pheromone itself.

Accordingly the present invention comprises the use in mosquito control of a compound of formula (II)

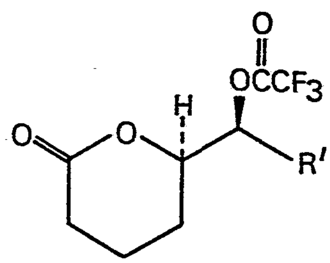

(II)

in which R' represents an alkyl group of 6 to 12 carbon atoms.

Such compounds (II), in which the acetoxy group of the natural pheromone compounds is replaced by a trifluoroacetoxy group, have certain advantages over the compounds (I) as mosquito attractants, as discussed in more detail hereinafter.

The alkyl groups R' may be straight or branched chain, although the former are of most interest. As indicated previously, the mosquito pheromones themselves contain a group R' of 8 or 10 carbon atoms and the compounds (II) which are preferred for use in compositions according to the present invention are those in which R' is a $C_{8-10}$ alkyl group, particularly an n-octyl and especially an n-decyl group. Compounds containing an alkyl group of 6 carbon atoms may however be of value in some contexts in view of their increased volatility, just as the use of an alkyl group of 12 carbon atoms leads to a decrease in volatility which may be of value, for example, in a long acting composition.

Formula (II) shows both the structure and the stereochemistry of the trifluoro compounds of use in the present invention, the compound being of the erythro as opposed to the threo configuration and being the 5R,6S rather than the 5S,6R enantiomer. Thus, it has now been found that, of the two erythro isomers of 6-acetoxy-5-hexadecanolide illustrated in UK Patent No. 2111841, it is the (-)-(5R,6S) isomer which corresponds to the natural pheromone and in a synthetic mixture of the (-)-(5R,6S) and (+)-(5S,6R) compounds, such as is described in the patent, the activity resides essentially in the former compound. Moreover, a similar situation has been found to apply with the trifluoroacetoxy compounds, although it should be pointed out that the presence of the other erythro isomer was not found to inhibit the expression of activity by the 5R,6S isomer. In practice, therefore, since the racemate containing both the 5R,6S and the 5S,6R enantiomer itself shows a good level of activity and, since it is simpler to prepare than the optically active 5R,6S isomer substantially free from the 5S,6R isomer, this dl-racemate or racemic mixture may often therefore be the material of choice rather than such a substantially pure isomer.

Specific compounds of use in the present invention are (5R,6S)-6-trifluoroacetoxy-5-dodecanolide, (5R,6S)-6-trifluoroacetoxy-5-tetradecanolide, (5R,6S)-6-trifluoroacetoxy-5-hexadecanolide and (5R,6S)-6-trifluoroacetoxy-5-octadecanolide.

Various routes are available to the compounds (II) but one convenient starting material is the corresponding 5,6-dihydroxyalkanoic acid of formula

$$HO_2C-(CH_2)_3-CH(OH)-CH(OH)-R' \qquad (III)$$

Such an acid may be reacted with any one of trifluoroacetic acid in the presence of a compound such as dicyclohexylcarbodiimide, trifluoroacetic anhydride and trifluoroacetyl chloride in the presence of a base such as pyridine to effect both trifluoroacetylation and lactonisation. As an alternative to the formation of the

compound (II) from a compound (III), the corresponding compound of formula (I) in which R is hydrogen may be trifluoroacetylated, for example using similar reagents, particularly the anhydride, to those used with the acid (III), or the corresponding compound of formula (I) in which R is acetyl may be subjected to acidolysis, for example by heating the acetoxy compound with trifluoroacetic acid in the presence of a catalyst such as p-toluene sulphonic acid, thereby replacing the acetyl group by a trifluoroacetyl group. The naturally occurring compounds of formula (I) in which R is acetyl (or hydrogen) and R' is n-octyl or n-decyl may be used as the starting material in such reactions but it is in practice much more convenient to use synthetic compounds (I). Since such synthetic compounds (I) are themselves most conveniently obtained from the corresponding acid (III), it will be appreciated that a procedure involving the conversion of the acid (III) to the compound (I) and then to the compound (II) has advantage over the direct route from (III) to (II).

In order to obtain the compound (I) as the 5R,6S isomer it is necessary to use a precursor having the appropriate configuration. For instance, the erythro dihydroxy acid (III) may be used, this conveniently being obtained, for example, by hydroxylation under suitable conditions of the appropriate (Z) or (E) unsaturated acid. Thus, the erythro isomer may be obtained by the cis hydroxylation, for example with potassium permanganate, of the (Z)-unsaturated acid or by the trans hydroxylation, for example with hydrogen peroxide and an organic acid such as formic acid, of the (E)-unsaturated acid. If the 5R,6S isomer is required substantially free from the 5S,6R isomer, a resolution step is introduced into the synthesis or a stereoselective synthesis is employed.

Although there are procedures available which may be used for the separation of a racemic mixture of the compounds (II) into separate enantiomers, for example preparative chiral gas chromatography using a substrate such as O-(1R,3R)-chrysanthemoyl-(S)-mandelic acid (R)-1-(1'-napthyl)-ethylamide or O-(1S,3S)-chrysanthemoyl-(R)-mandelic acid (S)-1-(1'-napthyl)-ethylamide, the

resolution is more conveniently effected at a suitable stage prior to formation of the compound (II) if significant amounts of the separate enantiomers are required. One convenient approach involves the preparation of a diastereoisomeric mixture of a compound containing a suitably modified group R from the substantially pure erythro form of the compound (I) in which the desired group R' is present and which has a group R which is hydrogen. Such a diastereoisomeric mixture of a suitable compound may be readily obtained by treatment of the compound (I) having R=H with a pure isomer of an optically active esterifying agent such as l- or particulaly d-α-methoxy-α-trifluoromethylphenylacetyl chloride. The diastereoisomers may then be separated, for example by thin layer chromatography or high pressure liquid chromatography, and the appropriate isomer then hydrolysed to yield the required 5R,6S compound (I) in which R is hydrogen and which may then be trifluoroacetylated to give the desired compound (II). An alternative approach involves the acidolysis of a compound (I) in which R' is the desired group and R is acetyl by treatment with a pure isomer of an optically active acid such as l or particularly d-α-methoxy-α-trifluoromethylphenylacetic acid to give a diastereoisomeric mixture which may be similarly separated. Acidolysis will then yield the required 5R,6S optically pure compound (II) in which R is trifluoroacetyl.

A procedure of particular value, however, for the preparation of the (5R,6S) isomer in substantially optically pure form utilises a stereoselective synthesis of the corresponding isomer of the equivalent compound (III), which can be obtained in a high level of optical purity by a route which commences with the epoxidation of the appropriate unsaturated alcohol, for example dl-1-tridecen-3-ol, by the Sharpless asymmetric epoxidation technique. The conversion of the optically pure compound (III) to the compound (II) may be effected via the compound (I) having R = acetyl or hydrogen or more preferably directly.

The routes to the compounds (II) from intermediates (III) or (I) which are described above, like the routes described in UK Patent No. 2111481 to the compounds (I) in which R is acetyl or hydrogen, are particularly adapted to the preparation of a (5R,6S)/(5S,6R) racemate substantially free from the two threo isomers, or of the 5R,6S isomer substantially free from the 5S,6R isomer. In particular, the synthesis of 6-hydroxy-5-hexadecanolide which is reported in that patent yields the dl-erythro compound substantially free from the threo isomer. We have now developed a novel and much simpler route to the intermediates (I) in which R is hydrogen which does, however, produce an admixture of the erythro and threo isomers, the intermediate therefore producing a similar mixture of isomers when converted to the compound (II). Rather surprisingly, it has been found that the presence of the threo isomers does not in any way inhibit the expression of activity by the 5R,6S isomer. Thus, the erythro/threo mixture shows an equally high level of activity, in relation to the amount of the 5R,6S isomer present, as does the pure isomer, the threo isomers apparently acting essentially as only an inert diluent of the 5R,6S isomer. Accordingly, although it would be possible with this route to effect an isomer separation as between the erythro and threo isomers and also between the two erythro isomers, the simplicity of the route is such that the mixture of erythro and threo isomers of a compound (II) obtained from the intermediate (I) obtained by the route is the material of choice for mosquito control in the field. However, it is possible, as described hereinafter to vary the reagent used at one point in the route to enhance the proportion of the erythro racemate relative to the threo racemate in the intermediate (I) to greater than 50% thereby providing an increase in the activity on a unit weight basis of the mixture of isomers of the compound (II) produced from this intermediate.

The present invention therefore further includes a process for the preparation of a compound of formula (Ia)

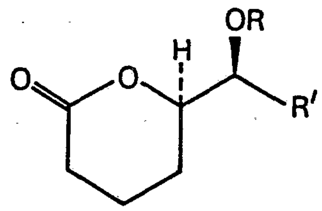

(Ia)

in which R represents hydrogen and R' represents an alkyl group of 6 to 12 carbon atoms which comprises effecting a ring expansion of a compound of formula (IV)

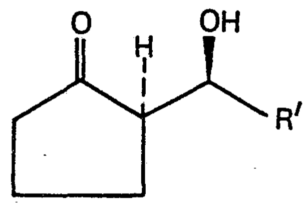

(IV)

in which R' represents the same alkyl group of 6 to 12 carbon atoms, through the insertion of an oxygen atom between the carbon atoms of the groupings -CO- and $>$CH-CH(OH)R' .

The insertion of the oxygen atom is most conveniently effected using a Baeyer-Villiger rearrangement utilising a peroxy compound, for example a peracid, which may be aliphatic such as peracetic acid or perchloroacetic acid or aromatic such as perbenzoic acid or particularly m-chloroperbenzoic acid. The peracids may either be used as such or formed in situ by the action of hydrogen peroxide on the corresponding organic acid. Where appropriate, for example particularly in the case of peroxy compounds other than peracids, an acid catalyst may be used, for example a Lewis acid such as aluminium chloride, boron trifluoride, stannic chloride or titanium tetrachloride. The reaction may conveniently be carried out in any suitable solvent, for example dichloromethane, and at a room temperature, cooling being used to control the resulting exothermic

reaction. Working up of the reaction mixture obtained using a peracid such as m-chloroperbenzoic acid may conveniently be assisted by the use of a complexing agent, particularly potassium fluoride, to precipitate the acid formed and any unreacted peracid. It has been found that the Baeyer-Villiger rearrangement substantially effects the insertion, as required, between the carbon atoms of the groupings -CO- and $>$CH-CH(OH)R' and not between those of the groupings -CO- and $-CH_2-$.

The compound (IV) may conveniently be prepared from cyclopentanone by means of aldol condensation reaction with the appropriate aldehyde R'CHO. The aldol condensation may conveniently employ either an ether derivative or a metal oxide derivative of the enol form of cyclopentanone. Thus, for example, the trimethylsilyl ether may be used, conveniently in the presence of a catalyst such as a Lewis acid, or more preferably an alkali metal enolate, for example the magnesium or lithium enolate, may be used. The value of these metal compounds lies in the isomeric nature of the product obtained in the aldol condensation. Thus, with the trimethylsilyl ether the resultant 2-(1-hydroxyalkyl)-cyclopentan-1-one is obtained as a mixture of essentially equal proportions of the erythro and threo isomers, whilst with the lithium enolate the proportion is between 3:1 and 4:1 in favour of the erythro isomer. It has been found that this proportion is maintained through the subsequent stages of the preparation so that an enhanced proportion of the erythro racemate relative to the threo racemate is obtained in the intermediate (I) and in the final product (II).

The compound (I) obtained from the compound (IV) may conveniently be trifluoroacetylated as described hereinbefore, for example with trifluoroacetyl chloride or particularly trifluoroacetic anhydride.

It will be appreciated that compounds (II) are conveniently prepared in a form substantially free from by-products of manufacture not having the formulae (I) or (II) shown hereinbefore.

The compounds (II) may be used in the present invention in the form of a composition containing more than one such compound of formula (II) in which R' differs, or even in admixture with one or more compounds of formula (I). However, in practice it is also preferable for the compounds (II) initially to be prepared in a form which is substantially free from by-products of manufacture which are compounds of formula (I), and preferably also from those which are different compounds (II), and for any mixture which is used to be produced by the deliberate mixing of selected proportions of the desired components, particularly of different compounds (II). The words "substantially free from" have been used herein particularly to indicate an amount of no more than 10% by weight of the specified contaminant(s).

It will be appreciated that the compounds described herein may also be prepared by other procedures than those described, particularly by other procedures described in the art for the preparation of related compounds of a similar type.

The compounds (II) may be formulated by various procedures to provide a composition of a form suitable for use in insect control, but these procedures will most usually involve combination with a suitable diluent or carrier, both liquid and solid formulations being of interest.

The present invention thus includes a composition for use in insect control which comprises as an active ingredient thereof a compound of formula (II) as defined hereinbefore together with a suitable diluent or carrier therefor.

Liquid compositions may incorporate a variety of inert liquids as the diluent or carrier, for example consisting of an oil based formulation or an aqueous formulation, which latter may conveniently contain a suitable amount, for example up to about 20%, particularly about 10%, by volume of an emulsifying agent, especially a non-ionic surface active agent which may conveniently be one based on a polyether structure, for example polyoxyethylene stearate or nonylphenylpolyethoxyethanol. The use of a surface active agent will promote contact of the composition with the site

to which it is applied but care should be taken to avoid unduly solubilising the compound (II). Other components may also be included in the composition and, although many of the compounds (II) have a very low solubility in water, it may be advantageous to include an agent such as a silicone oil in the composition to further prevent mixing of the composition with the water usually present at the location to which it is applied. The compounds may also be applied in the field as monolayers or by the use of various other techniques known in the art. Formulations for domestic use, for example as aerosols, are also of some interest.

Solid compositions may incorporate a variety of inert solids as the diluent or carrier and may take various forms, for example microcapsules, but one form which is of some particular interest involves the formulation of the compound (II) as a tablet which effervesces on contact with water. Such effervescent tablets may be prepared using conventional solid carrier materials, for example a sodium bicarbonate/tartaric acid/starch mixture, to which the compound may conveniently be added in a small amount of a liquid diluent, for example one of a hydrocarbon nature. Tablet formation may then be effected by compression in the conventional manner.

Techniques such as electrostatic spraying may of course be used for applying the composition, where appropriate, and may even enable the compound (II) to be applied without the addition of a diluent or carrier. The present invention thus includes an insect control composition comprising a compound of formula (II) as defined hereinbefore as an active ingredient thereof, said compound (II) being in the form of a charged dispersion. Such forms of composition are well described in the art in other contexts but the following guidance may be given. With electrostatic spraying using the Rothamsted equipment (described in UK Patent No. 2073053, and subsequently in UK Patent 2081133 and UK Patent Application No. 2119678) the compound may conveniently be in the form of aqueous emulsion or a solution in an inert solvent, the formulation preferably having a conductivity which lies in a range of $1 \times 10^{-2}$ to $1 \times 10^{-9}$ ohms/cm. A suitable conductivity is

usually achieved by the addition of an appropriate agent, such as the Shell product ASA3. With the ICI Electrodyne equipment, the compound (II) may conveniently be in the form of a solution in an organic solvent, the formulation preferably having a conductivity of about $1 \times 10^{-8}$ ohms/cm.

The value of the compositions according to the present invention lies in their use in the attraction of gravid female mosquitoes of the genus Culex, and also of certain other genera, especially those related genera of egg raft laying mosquitoes. Such mosquitoes are conveniently attracted to particular sites which are further treated previously, concommitantly or subsequently to kill the young mosquitoes in the egg or larval form and/or, more particularly, to kill the adult, particularly the adult female, mosquitoes. Mosquitoes generally lay their eggs on water and the compounds are therefore conveniently applied in the vicinity of or preferably directly on the surface of areas of water where the mosquitoes may be expected to breed naturally, for example water in proximity to human habitation such as is present in artificial containers, tanks, drains, cess pits etc., or alternatively to artificially created areas of water, for example water contained in conveniently positioned receptacles. A specific example of such a use is the placing of an effervescent tablet, as described hereinbefore, in a bucket or a bowl when it releases the compound (II) which rises to the surface of the water and attracts mosquitoes. Such a method may be applied with particular advantage to domestic habitats where there is close association between the disease vector and man. The invention thus includes a method for use in mosquito control which comprises applying a compound (II) as defined hereinbefore to a location for the purpose of attracting mosquitoes to that location. Mosquitoes attracted to the location by the compound (II) and/or their eggs or larvae may then be destroyed.

Although the mosquitoes, once attacted, may be destroyed by physical means, for example by trapping at the location to which the compound (II) is applied, it is often more convenient to use

some form of insecticidal agent to destroy the eggs, larvae or adult mosquiteos. A wide variety of agents may be used for this purpose either in the bulk of water at the site of application or on the surface thereof, including inorganic poisons and even the production of a high salt level in the water. Organic pesticides are, however, of especial interest, particularly pyrethroid pesticides, for example permethrin, organophosphorus pesticides, for example malathion or fenitrothion and some carbamates, for example carbaryl, etc. Compounds of particular value as ovicides or larvicides, such as chlorpyrifos, temephos, diflubenzuran, chlorodimeform, growth regulators such as methoprene and dimilin, pathogenic organisms, such as Bacillus thuringiensis, or their toxins may be utilised, for example as larvicidal pellets, dusts or oils, either alone or together with insecticides such as those described above which are of particular value for use against adult mosquitoes. The insecticides may be applied previously or subsequently to the attractant compound (II) in a separate procedure but it is particularly convenient for the insecticide or insecticides to be applied together with this compound. An especially convenient aspect of the present invention consists of a mosquito control composition comprising as active components thereof a compound (II) as defined hereinbefore together with an insecticide.

Whilst quantitative details of the use of the compounds (II) will depend on various factors, not least the particular compound (II) employed and its isomeric purity, it is an indication of the level of activity of these compounds that in laboratory tests, which are described hereinafter in Example 6, synthetic (±)-erythro-6-trifluoroacetoxy-5-hexadecanolide has been shown to act as strong attractant for gravid Culex mosquitoes at levels as low as 3 micrograms. In practice, however, although activity at very low levels has been observed in the laboratory, it is usually desirable in the field to employ a larger amount of 6-trifluoroacetoxy-5-hexadecanolide, or of the equivalent amount of another

compound (II). For example, an amount of 25, 50 or 100 micrograms up to 1 mg or more of compound per week may be applied, for example as a slow release formulation or in effervescent tablet form, with due attention being given to the size of the site to which the application is made. Thus, a natural site containing a large surface area of water will require larger amounts of compound than a smaller artificial receptacle, the amount required in the former instance possibly being several milligrams or tens of milligrams.

The compositions of the present invention provide a useful alternative to those of UK Patent No. 2111481 and may provide one or more of a number of advantages thereover depending upon the particular use to which the composition is applied. In particular, the activity of the compositions of the present invention is, in general, at least equal to that of the compositions containing the unfluorinated compounds and for certain strains of mosquito, at least, it may be significantly higher. Apart from the question of their basic level of activity, however, the compositions of the present invention have properties which can be particularly advantageous to their use in the field, such as greater volatility (the fluorinated compounds have a significantly shorter half life on volatilisation from glass) which provides a wider range of attractant effect, possibly greater resistance to degradation, etc.

The invention is illustrated by the following examples.

## EXAMPLES

### Example 1

**Preparation of *erythro*-6-trifluoroacetoxy-5-hexadecanolide**

*erythro*-5,6-Dihydroxyhexadecanoic acid (320 mg, 1.1 m.mole) is treated with trifluoroacetic anhydride (2 ml) and the reaction mixture is allowed to stand at room temperature for 4 hours. The excess trifluoroacetic anhydride is then evaporated under nitrogen and the resdiual oil is dissolved in hexane (5 ml), the solution being washed with water (4 x 5 ml), dried over $MgSO_4.H_2O$ overnight, filtered and evaporated to yield *erythro*-6-trifluoroacetoxy-5-hexadecanolide as a colourless viscous oil (330 mg, 0.9 m.mole, 81%).

The procedure described above is applied to synthetic (±)-erythro-5,6-dihydroxyhexadecanoic acid, (+)-erythro-5,6-dihydroxyhexadecanoic acid and (-)-erythro-5,6-dihydroxyhexadecanoic acid, the (±) compound having been prepared as described in UK Patent No. 2111481 and the (+)-(5S,6R) and (-)-(5R,6S) compounds as described by Mori and Otsuka, Tetrahedron, 1983, 39, 3267. The resultant products, (±)-(5R,6S/5S,6R)-erythro-6-trifluoroacetoxy-5-hexadecanolide, (+)-(5S,6R)-6-trifluoroacetoxy-5-hexadecanolide and (-)-(5R,6S)-6-trifluoroacetoxy-5-hexadecanolide, are subjected to gas chromatography employing a 25 m x 0.3 mm i.d. OV101 bonded phase fused silica column (Scientific Glass Engineering) linked directly to a MM 70-70F mass spectrometer (V.G. Analytical) at 200°C and electron impact at 70 eV. The carrier gas is helium (235 mm/second) and the oven temperature is held initially at 50°C for 10 minutes and is then programmed at 4°C/minute to 200°C. Each of the isomeric products showed the same retention time, Rt = 70 minutes, with the following identical mass spectrometry characteristics: m/z 366 ($M^+$, 7%), 252 (14), 99 (100), 82 (52), 81 (70), 71 (94), 69 (67), 67 (68), 55 (90), 42 (56).

The (±)-erythro-6-trifluoroacetoxy-5-hexadecanolide is subjected to chiral chromatography using O-(1S,3S)-chrysanthemoyl-(R)-mandelic acid (S)-1-(1'-naphthyl)-ethylamide, which was prepared from (1S,3S)-chrysanthemic acid, (R)-mandelic acid and (S)-1-(1'-naphthyl)-ethylamine (Salford Ultrafine Chemicals and Research) by the method employed by Oi et al, Journal of Chromatography, 1983, 254, 282 for the alternative enantiomer. This material is used to prepare a 25 m x 0.22 mm i.d. fused silica capillary column (Chrompak) which is mounted in a Pye Series 104 gas chromatograph equipped with a flame ionisation detector and with helium (15 p.s.i.) as the carrier gas. Samples are introduced using direct on-column injection onto a cooled column (25°C) and after 20 seconds the oven temperature is ballistically programmed to 160°C, this being the maximum operating temperature of the column. Using this system the (±)-erythro compound showed almost baseline

separation into the (5S,6R) and (5R,6S) compounds, co-injection with the pure enantiomers indicating that the (5R,6S) enantiomer is eluted before the (5S,6R) enantiomer (Rt =218 and 222 minutes).

Example 2

Preparation of erythro/threo-6-trifluoroacetoxy-5-hexadecanolide

(A) Equal proportion of erythro and threo isomers

(1) 1-Trimethylsilyloxycyclopent-1-ene

A solution of cyclopentanone (8.4 g, 0.1M), potassium iodide (16.6 g, 0.1M) and triethylamine (11.0 g, 0.11M) in dimethylformamide (50 ml) is stirred and treated with trimethylsilychloride (TMS-Cl) (11.0 g, 0.1M) over 0.25 hours with water cooling. Further quantities of triethylamine (2.0 g) and TMS-CL (2.0 g) are added 1 hour later and the mixture is allowed to stir overnight. The mixture is then partitioned by the addition of petroleum ether (60:80 fraction, 100 ml) and water (100 ml), the organic phase being washed with water (3 x 100 ml). After drying, the petroleum ether is removed and the residue distilled at water pump pressure to give the title compound as a colourless liquid (12.0 g, 77%), b.p. 60-61$^{o}$C/25 torr, $^{1}$Hnmr (60 MHz), 0.14 (s, 9H), 1.80-2.40 (m, 6H), 4.45 (br.t, 1H).

(2) erythro/threo-2-(1-Hydroxyundecyl)-cyclopentan-1-one

Titanium tetrachloride (9.5 g, 0.05M) in dichloromethane (40 ml) is stirred at -78$^{o}$C under nitrogen and treated dropwise during 0.25 hours with a mixture of the 1-trimethylsilyloxycyclopent-1-ene (7.87 g, 0.05M) and undecan-1-al (8.5 g, 0.05M). Stirring is continued for a further 0.5 hours, and water (50 ml) is then added and the mixture partitioned by the addition of petroleum ether (60:80 fraction, 100 ml) and water (100 ml). The organic layer is washed with water (3 x 50 ml) and saturated sodium bicarbonate (70 ml), and dried. The petroleum ether is then evaporated to give the title compounds as the residue (13.3 g), $^{13}$Cnmr (100 MHz): (erythro) 14.14, 20.63, 22.75, 24.94, 26.76, 29.73(5), 31.98, 35.19, 38.47, 53.88, 72.20, 223.40;

(threo) 14.14, 20.75, 22.75(2), 26.15, 29.73(5), 32.03, 35.13, 39.19, 54.48, 69.65, 221.64 (the proportions being equal).

(3) erythro/threo-6-Hydroxy-5-hexadecanolide

The crude 2-(1-hydroxyundecyl)-cyclopentan-1-one (13.3 g) is dissolved in dichloromethane (100 ml) and treated with m-chloroperbenzoic acid (85%, 10 g, 0.05M) using water cooling, when an exothermic reaction occurs. After 3 hours stirring, potassium fluoride (3.0 g, 0.05M) is added and the resulting insoluble complexes are removed by filtration. The solid is washed with dichloromethane (100 ml) and the filtrate and washings are concentrated. The residue is dissolved in hexane (100 ml), the precipitate (10.75 g) which forms is removed by filtration and a further precipitate (0.85 g) produced by reducing the volume of the mother liquors, the combined solids providing the title compound (11.6 g, 86% overall yield from the 1-trimethylsiloxy-cyclopent-1-ene), $^{13}$Cnmr (100 MHz): (erythro) 14.14, 18.50, 22.75, 24.21, 25.60, 29.67(6), 31.8, 32.7, 73.30, 83.3, 171.95; (threo) 14.14, 18.50, 21.42, 22.75, 25.91, 29.67(6), 31.9(2), 72.38, 83.61, 171.70 (the proportions being equal).

(4) erythro/threo-6-trifluoroacetoxy-5-hexadecanolide

The 6-hydroxy-5-hexadecanolide (5.0 g) is treated with trifluoroacetic anhydride (10 g) and the mixture allowed to stand at room temperature for 1 hour. The volatile materials are removed under reduced pressure using added aliquots of toluene until the title compound is obtained as a residue of constant weight (6.7 g, 100%), $^{13}$Cnmr (100 MHz) (erythro and threo: not assigned) 14.14, 17.90, 22.40 + 23.97, 22.87, 24.94 + 25.16, 29.55(7), 32.16, 78.57, 81.30 + 81.48, 114.93(q, $CF_3$, J=281), 157.38 (q, CO-$CF_3$, J=40), 175.65.

(B) Enhanced proportion of erythro isomer

(1) erythro/threo-1-(1-Hydroxyundecyl)-cyclopentan-1-ene

Diisopropylamine (2.52 g, 0.025M) in dimethoxyethane (10 ml) is treated at -15$^{\circ}$C under $N_2$ with butylithium (9.5M) in hexane (2.8 ml, 0.025M) and the mixture left for 0.5 hours. The mixture is then

treated at -78°C with cyclopentanone (2.1 g, 0.025M)[1] followed, after 0.5 hours, by undecan-1-al (4.25 g, 0.025M), the reaction then being left for a further 0.5 hours. Aqueous acetic acid is then added to pH 5, followed by water (100 ml) and petroleum ether (60:80 fraction, 50 ml) and the mixture partitioned. The organic phase is washed with water (2 x 50 ml), dried and concentrated to give the title compound as a clear oil (6.0 g). Analysis of this product by $^{13}$Cnmr indicates that the yield of the 2-(1-hydroxydecyl-cyclopentan-1-ene is approximately 65% of which 50% is the erythro isomer and 15% is the threo isomer.

(2) erythro/threo-6-Trifluoroacetoxy-5-hexadecanolide

The 2-(1-hydroxyundecyl)-cyclopentan-1-ene is converted to 6-hydroxy-5-hexadecanolide and then to 6-trifluoroacetoxy-5-hexadecanolide in an exactly similar manner to that described under sections (3) and (4) of (A).

The enhancement of the proportion of the erythro isomer in the 2-(1-hydroxyundecyl)-cyclopentan-1-ene, and the products derived therefrom, is confirmed by the treatment of the 6-hydroxy-5-hexadecanolide obtained from 3.0 g of this compound with hexane when it is possible to precipitate a solid product (0.9 g, 28%) containing a 1:1 proportion of the erythro/threo isomers and to isolate the pure erythro isomer from the mother liquors as a colourless oil (1.05 g, 32.7%).

Example 3

Preparation of erythro-6-trifluoroacetoxy-5-hexadecanolide from the apical droplets of mosquito eggs

Apical droplets from 20 egg rafts of Culex p. fatigans (corresponding to about 6 µg of erythro-5,6-dihydroxyhexadecanoic acid) are removed into hexane (about 500 µl) using a fine glass

---

[1] In a variation of this procedure the lithium enolate is prepared by the treatment of the trimethylsilyl enol ether, prepared as in section (1) of (A), with methyl-lithium.

rod. The hexane is removed under nitrogen and the residue is stored with 2M aqueous NaOH (100 μl) overnight. The solution is then acidified (2M aqueous HCl), extracted with ether (5 ml), dried over $MgSO_4.H_2O$ and the solvent evaporated under nitrogen. The residue is trifluoroacetylated using the procedure described under Example 1 and the product subjected to GC-MS. The gas chromatography showed only one major component corresponding to the (5R,6S) enantiomer obtained from the synthetic (5R,6S)-5,6-dihydroxyhexadecanoic acid, the mass spectrometry characteristics being identical to those of the synthetic compound.

Example 4

Preparation of erythro-6-trifluoroacetoxy-5-dodecanolide

(±)-erythro-5,6-Dihydroxydodecanoic acid (1 mg, prepared as described in Example 3 of UK Patent No. 2111841 is treated with trifluoroacetic anhydride (100 μl) and the reaction mixture is allowed to stand overnight at room temperature in a sealed ampoule. The excess trifluoroacetic anhydride is then evaporated under nitrogen and the residue subjected to gas chromatography mass spectrometry (GC-MS) to yield (±)-erythro-6-trifluoroacetoxy-5-dodecanolide. The gas chromatography employs a 25 m x 0.3 mm i.d. OV101 bonded phase fused silica column (Scientific Glass Engineering) linked directly to a MM 70-70F mass spectrometer (V.G. Analytical) at 200°C and electron impact at 70 eV. The carrier gas is helium (235 mm/second) and the oven temperature is held initially at 50°C for 10 minutes and is then programmed at 4°/minute to 200°C. The (±)-erythro-6-trifluoroacetoxy-5-dodecanolide reaction product shows a single main peak of retention time, Rt = 50 minutes with the following mass spectrometry characteristics: m/z 310 ($M^+$, 0.5%), 208 (14), 99 (100), 75 (99), 55 (96), 43 (69), 42 (72), 41 (64), 29 (79), 27 (87).

The (±)-erythro-6-trifluoroacetoxy-5-dodecanolide is subjected to chiral chromatography using an exactly similar procedure to that described in Example 1 for (±)-erythro-6-trifluoroacetoxy-5-hexadecanolide when a resolution is again

achieved into the (5R,6S) and (5R,6S) isomers, the two peaks having a retention time Rt = 86 and 88 minutes, respectively.

Example 5

Preparation of effervescent tablet formulation

To a mixture of sodium bicarbonate (7.5 g), tartaric acid (8.4 g) and starch (7.9 g) is added a mixture of 600 µl of hexane containing (±)-erythro-6-trifluoroacetoxy-5-hexadecanolide (6 mg) and liquid paraffin (36 mg). The resultant mixture is compressed in batches of 3.8 g to provide effervescent tablets each containing about 1 mg of the active compound.

Example 6

Tests of oviposition activity

The tests were carried out in a cage (30 cm x 30 cm x 30 cm) of gravid formulae mosquitoes [Culex pipiens fatigans (= quinquefasciatus); Lagos strain] in which were placed two bowls of 13 cm diameter containing water. In the first, test bowl was floated a 12 mm x 1 mm polystyrene disc to which 3 µg of (±)-erythro-6-trifluoroacetoxy-5-hexadecanolide (prepared as described in Example 1) in 4 µl hexane had been applied, whilst in the second, control bowl was floated an identical disc to which 4 µl of hexane had been applied. Ten replicate experiments were carried out, with the position of the test and control bowls being alternated for each successive replicate.

The number of egg rafts laid by the mosquitoes in the test and control bowls were counted and are given in the Table below. It will be seen that, of the total of egg rafts laid, 90.34% were laid in the test bowl (Student $t = 5.633$, $p < 0.001$). These results show an outstandingly consistent, strong attractancy by the (±)-erythro-6-trifluoroacetoxy-5-hexadecanolide.

TABLE

| Replicate experiment | Number of egg rafts laid | |
|---|---|---|
| | Test bowl | Control bowl |
| 1 | 43 | 1 |
| 2 | 19 | 5 |
| 3 | 25 | 2 |
| 4 | 62 | 6 |
| 5 | 40 | 2 |
| 6 | 28 | 8 |
| 7 | 6 | 0 |
| 8 | 14 | 1 |
| 9 | 20 | 3 |
| 10 | 33 | 1 |
| Total | 290 | 31 |

CLAIMS

1. An insect control composition comprising a compound of formula (II)

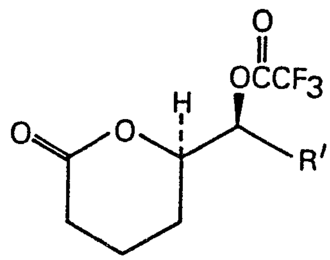

(II)

in which R' represents an alkyl group of 6 to 12 carbon atoms, together with a suitable diluent or carrier.

2. A composition according to Claim 1, in which the group R' in the compound (II) represents an alkyl group of 8 to 10 carbon atoms.

3. A composition according to Claim 1 or 2, in which the group R' in the compound (II) is a straight chain alkyl group.

4. A composition according to Claim 1, in which the group R' in the compound (II) is n-octyl or n-decyl.

5. A composition according to Claim 1, in which the compound (II) is (5R,6S)-6-trifluoroacetoxy-5-tetradecanolide.

6. A composition according to Claim 1, in which the compound (II) is (5R,6S)-6-trifluoroacetoxy-5-hexadecanolide.

7. A composition according to any of Claims 1 to 6, in which the compound (II) is in the form of the (5R,6S)/(5S,6R) racemate.

8. A composition according to Claim 7, in which the (5R,6S)/5S,6S is in admixture with the (5S,6S)/(5R,6R) racemate.

9. A composition according to any of Claims 1 to 8, which comprises an inert liquid as the diluent or carrier.

10. A composition according to Claim 9, which contains an emulsifying agent.

11. A composition according to any of Claims 1 to 8, which comprises an inert solid as the diluent or carrier.

12. A composition according to Claim 11 in microcapsule or effervescent tablet form.

13. An insect control composition comprising a compound of formula (II)

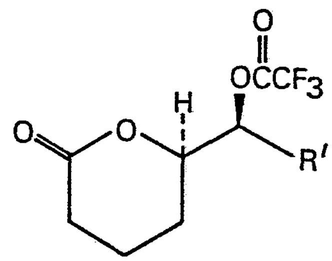

(II)

in which R' represents an alkyl group of 6 to 12 carbon atoms, in the form of a charged dispersion.

14. A composition according to Claim 13, in which the compound (II) is as defined in any of Claims 2 to 8.

15. A composition according to any of Claims 1 to 14, which comprises an insecticide as an additional active component.

16. The use of a compound of formula (II)

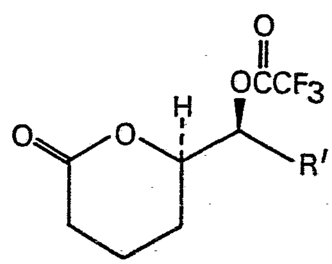

(II)

in which R' represents an alkyl group of 6 to 12 carbon atoms in the manufacture of a mosquito control agent.

17. The use in mosquito control of a compound of formula (II)

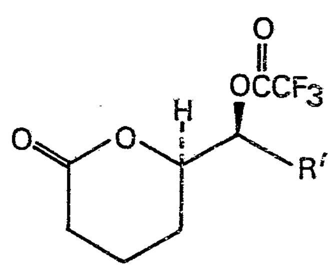

(II)

in which R' represents an alkyl group of 6 to 12 carbon atoms.

18. A use according to Claim 16 or 17, in which the compound (II) is as defined in any of Claims 2 to 8.

19. A method of mosquito control which comprises attracting mosquitoes to a location by applying thereto a compound of formula (II)

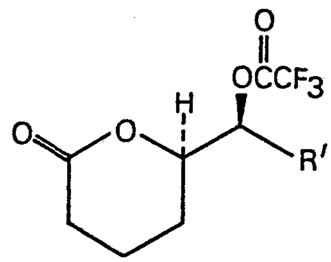

(II)

in which R' represents an alkyl group of 6 to 12 carbon atoms.

20. A method according to Claim 19, in which the compound is applied as a composition according to any of Claims 1 to 14.

21. A method according to Claim 19 or 20, in which the location is treated to destroy the mosquitoes attracted thereto and/or their eggs or larvae.

22. A method according to Claim 21, in which the mosquitoes and/or eggs or larvae are destroyed with an insecticide.

23. A method according to Claim 22, in which the location is treated with a composition according to Claim 15.

24. A process for the preparation of a compound of formula (II)

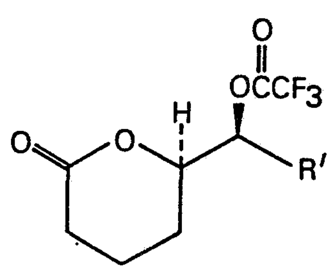

(II)

in which R' represents an alkyl group of 6 to 12 carbon atoms, which comprises trifluoroacetylating a compound of formula (Ia)

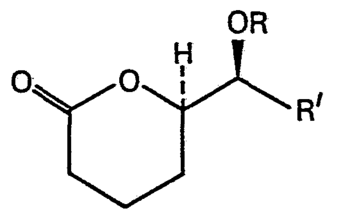

(Ia)

in which R represents hydrogen and R' represents the same alkyl group of 6 to 12 carbon atoms.

25. A process according to Claim 24, in which the trifluoroacetylation is effected with trifluoroacetic anhydride.

26. A process according to Claim 24 or 25, in which the compound (II) is as defined in any of Claims 2 to 8.

27. A process for the preparation of a compound of formula (Ia)

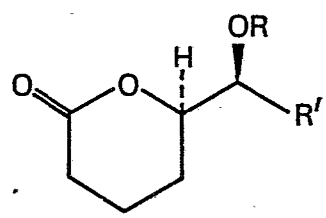

(Ia)

in which R represents hydrogen and R' represents an alkyl group of 6 to 12 carbon atoms, which comprises effecting a ring expansion of a compound of formula (IV)

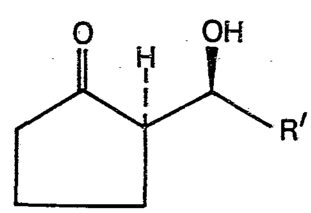

(IV)

in which R' represents the same alkyl group of 6 to 12 carbon atoms, through the insertion of an oxygen atom between the carbon atoms of the groupings -CO- and >CH-CH(OH)R' .

28. A process according to Claim 27, in which the ring expansion is effected through reaction with a peroxy compound.

29. A process according to Claim 28, in which the reaction is effected with a peracid.

30. A process according to Claim 27, 28 or 29, in which the group $R^V$ is as defined for the compound (II) in any of Claims 2 to 4.

31. A process according to any of Claims 27 to 30, in which the compounds (Ia) and (IV) are each in the form of the (5R,6S)/(5S,6R) racemate which is in admixture with the (5S,6S)/(5R,6R) racemate.

0194077

European Patent Office

# EUROPEAN SEARCH REPORT

Application number

EP 86 30 1205

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| X | "The Merck index", 1983, 10th edition, section: organic name reactions, pages 6-7, Merck & Co., Inc., Rahway, N.J., US * Baeyer-Villiger reactions * --- | 27-31 | A 01 N 43/16<br>C 07 D 309/30 |
| A | EP-A-0 078 641 (NATIONAL RESEARCH DEVELOPMENT) & GB - A - 2 111 481 (Cat. D) ----- | 1-31 | |
| | | | TECHNICAL FIELDS SEARCHED (Int. Cl.4) |
| | | | A 01 N<br>C 07 D |

The present search report has been drawn up for all claims

| Place of search | Date of completion of the search | Examiner |
|---|---|---|
| THE HAGUE | 27-05-1986 | DECORTE D. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document